# EUROPÄISCHE PATENTSCHRIFT

(11) **EP 0 406 657 B1**
(45) Veröffentlichungstag und Bekanntmachung des Hinweises auf die Patenterteilung: **31.08.1994**
(21) Anmeldenummer: 90111997.4
(22) Anmeldetag: 25.06.1990
(51) Int. Cl.: C09C 1/00, C08K 7/26, C08K 13/04, A61K 7/027, A61K 7/035

(54) **Plättchenförmige Substrate**
Platelets as substrates
Substrats en paillettes

(30) Priorität: 06.07.1989 DE 3922178
(43) Veröffentlichungstag der Anmeldung: 09.01.1991
(73) Patentinhaber: MERCK PATENT GmbH, D-64271 Darmstadt (DE)
(72) Erfinder: Prengel, Constanze, Dr., D-6108 Weiterstadt (DE); Dietz, Johann, Dr., D-6057 Dietzenbach (DE); Thurn-Müller, Angelika, Dr., D-6109 Mühltal 4 (DE)

(56) Entgegenhaltungen:
- JP-A-60 255 712
- US-A- 512 226
- US-A- 3 022 185
- US-A- 4 072 537
- US-A- 4 603 153

## Beschreibung

Die Erfindung betrifft deagglomerierte und gut dispergierbare plättchenförmige Substrate mit hohem Weichheitsgrad.

Plättchenförmige Substrate, d.h. Materialien mit einer im Vergleich zu Länge und Breite relativ geringen Dicke, werden in vielen Bereichen der Technik eingesetzt. So werden z. B. plättchenförmige Mineralien, wie z. B. Glimmer oder Talk, in feinteiliger Form als Füller für Kunststoffe eingesetzt. Plättchenförmige Pigmente, wie z. B. mit Metalloxiden beschichtete Glimmerplättchen oder plättchenförmiges Eisenoxid, finden sowohl zur Pigmentierung von z. B. Lacken, Farben, Kunststoffen und dergleichen als auch in kosmetischen Zübereitungen Verwendung.

Ein grundsätzliches Problem ist die starke Neigung von solchen plättchenförmigen Substraten zur Bildung von Agglomeraten, in denen die Substrate stapelförmig aufeinander liegen und aufgrund von starker Adhäsion nur schwer wieder zu separieren sind. Dies ist um so schwerwiegender als bei der Einarbeitung von plättchenförmigen Substraten in Zubereitungen wegen der leichten Zerstörbarkeit der dünnen Substrate keine hohen Scherkräfte ausgeübt werden dürfen.

Zur Verhinderung von Agglomeration, die sich sowohl durch eine ungleichmäßige Pigmentierung der Zubereitungen als auch bei Kosmetika durch ein unangenehmes Hautfeeling bemerkbar macht, wurden bisher insbesondere chemische Beschichtungen der Oberfläche solcher Substrate vorgeschlagen. Dies kann jedoch insbesondere bei plättchenförmigen Farbglanzpigmenten zu einer unerwünschten Beeinträchtigung der optischen Eigenschaften führen. In vielen Fällen führen jedoch auch solche Methoden nicht zum Erfolg, so daß solche Substrate nur in Form von Suspensionen, nicht jedoch in trockener Form angeboten werden können.

Es bestand deshalb nach wie vor die Aufgabe, die Agglomeratbildung von in trockener Form gehandhabten plättchenförmigen Substraten zu verhindern und gut dispergierbare Substrate mit ggf. angenehmem Hautfeeling bereitrustellen.

Es wurde nun überraschenderweise gefunden, daß diese Aufgabe dadurch gelöst werden kann, daß das plättchenförmige Substrat in dispergierter Form mit weitgehend sphärischen Teilchen vermischt wird, wodurch nach dem Trocknen der Suspension ein deagglomeriertes weiches Substratpulver erhalten wird.

Gegenstand der Erfindung sind daher deagglomerierte und gut dispergierbare plättchenförmige Substrate mit hohem Weichheitsgrad, gekennzeichnet durch einen Gehalt von mindestens 0,5 Gew.% sphärischer Teilchen mit im Vergleich zum plättchenförmigen Substrat kleinem Durchmesser, wobei das Verhältnis mittlerer Durchmesser der plättchenförmigen Partikel zu mittlerem Durchmesser der sphärischen Teilchen 10 : 1 oder mehr beträgt.

Gegenstand der Erfindung ist auch ein Verfahren zur Herstellung von deagglomerierten und gut dispergierbaren plättchenförmigen Substraten mit hoher Weichheit, das dadurch gekennzeichnet ist, daß man eine Suspension eines plättchenformigen Substrats mit einer Suspension sphärischer Teilchen mit im Vergleich zum plättchenförmigen Substrat kleinem Durchmesser vermischt und daraus das Substrat durch abfiltrieren und trocknen gewinnt.

Weiterhin ist Gegenstand der Erfindung die Verwendung solcher Substrate in technischen oder kosmetischen Formulierungen.

Offenbar wird durch die Zumischung der sphärischen Teilchen verhindert, daß die plättchenförmigen Substrate sich in nennenswertem Umfang aufeinander legen und damit eine starke Adhäsion ausüben können. Wie elektronenmikroskopische Aufnahmen zeigen, lagern sich die sphärischen Teilchen an der Oberfläche des plättchenförmigen Substrats an und ermöglichen daher wie eine Art Kugellager die leichte Verschiebbarkeit der Substratplättchen gegeneinander.

Um in diesem Sinne optimal wirken zu können, sollten die sphärischen Teilchen einen im Vergleich zum plättchenförmigen Substrat kleinen Durchmesser aufweisen. Da insbesondere plättchenförmige Substrate mit einem größten Durchmesser von etwa 1-500 µm in Frage kommen, sind sphärische Materialien mit einem Durchmesser im Bereich von etwa 0,05-50 µm bevorzugt.

In JP 60-255.712 werden kosmetische Präparate beansprucht. welche plättchenförmige Substrate und mehr oder weniger sphärische Teilchen enthalten. Die Präparate werden dabei durch Mischen zweier Komponenten A und B erhalten, wobei Komponente B das plättchenförmige Substrat, die sphärischen Partikel und weitere farbige Pigmente enthält. Die sphärischen Teilchen weisen jedoch einen ziemlich großen Durchmesser von typischerweise 10 µm auf, und das Verhältnis aus dem Durchmesser der plättchenförmigen Substrate zum Durchmesser der sphärischen Partikel beträgt zwischen 0.5 und 3.

Gegenstand der Erfindung ist auch ein Verfahren zur Herstellung von deagglomerierten und gut dispergierbaren plättchenförmigen Substraten mit hoher Weichheit, das dadurch gekennzeichnet ist, daß man eine Suspension eines plättchenförmigen Substrats mit einer Suspension sphärischer Teilchen mit im Vergleich zum plättchenformigen Substrat kleinem Durchmesser vermischt und daraus das Substrat durch abfiltrieren und trocknen gewinnt.

Weiterhin ist Gegenstand der Erfindung die Verwendung solcher Substrate in technischen oder kosmetischen Formulierungen.

Offenbar wird durch die Zumischung der sphärischen Teilchen verhindert, daß die plättchenförmigen Substrate sich in nennenswertem Umfang aufeinander legen und damit eine starke Adhäsion ausüben können. Wie elektronenmikroskopische Aufnahmen zeigen, lagern sich die sphärischen Teilchen an der Oberfläche des plättchenförmigen Substrats an und ermöglichen daher wie eine Art Kugellager die leichte Verschiebbarkeit der Substratplättchen gegeneinander.

Um in diesem Sinne optimal wirken zu können, sollten die sphärischen Teilchen einen im Vergleich zum plättchenförmigen Substrat kleinen Durchmesser aufweisen. Da insbesondere plättchenförmige Substrate mit einem größten Durchmesser von etwa 1-500 µm in Frage kommen. sind sphärische Materialien mit einem Durchmesser im Bereich von etwa 0,05 - 50 µm bevorzugt.

Geeignete sphärische Teilchen sind insbesondere anorganische Materialien, wie z. B. SiO₂, TiO₂ und ZrO₂. Sphärische Teilchen auf Basis von SiO₂ in einem Teilchengrößenbereich von etwa 3-15 µm sind z. B. als Materialien für die Hochdruckflüssigkeitschromatographie bekannt und werden z. B. als LiChrospher® von der E. Merck, Darmstadt vertrieben.

Besonders bevorzugt werden jedoch noch feinteiligere Materialien eingesetzt, insbesondere Materialien mit einer Teilchengröße im Bereich von etwa 0,05-1 µm. Vorzugsweise werden solche Teilchen in monodisperser Form, d.h. mit einer möglichst einheitlichen Teilchengröße eingesetzt. Bekannt sind solche monodispersen sphärischen Teilchen auf Basis von SiO₂, TiO₂ und ZrO. Monodisperses SiO₂ kann beispielsweise nach der DE-A1-36 16 133 hergestellt werden, die entsprechenden Materialien auch auf Basis TiO₂ und ZrO₂, sind jedoch auch als Dispersionen im Handel erhältlich.

Die verbesserte Deagglomeration der Substratteilchen läßt sich sehr einfach sensorisch durch Aufstreichen des trockenen Substrates auf die Haut durch die dabei zu spürende verbesserte Weichheit des Substratpulvers feststellen. Überraschenderweise zeigt es sich dabei, daß bereits mit sehr geringen Mengen der sphärischen Materialien deutliche Verbesserungen zu erzielen sind. So wird bereits mit etwa 0,5 Gew.% eine deutliche Verbesserung erzielt. Hervorragende Ergebnisse erzielt man schon bei Zumischung von etwa 1,5-3 Gew.% des sphärischen Materials. In der Regel setzt man daher Mengen von etwa 0,5-20 Gew.%, vorzugsweise etwa 1-10 Gew.%, ein. Größere Mengen lassen sich problemlos mit dem Substrat mischen, es wird dabei jedoch in der Regel keine weitere Verbesserung der Weichheit erreicht.

Wie bereits erwähnt, kann mit dem erfindungsgemäßen Zusatz von sphärischen Teilchen die Dispergierbarkeit und die Weichheit von beliebigen plättchenförmigen Substraten verbessert werden. In der Regel besitzen solche Substrate einen Durchmesser von etwa 1-500 µm, insbesondere etwa 5-200 µm, und eine Dicke von etwa 0,05-5 µm, insbesondere etwa 0,1-1 µm. Als Materialien können insbesondere genannt werden: Plättchenförmige Zeolithe wie Glimmer oder Talk, Glas, plättchenförmiges Eisenoxid, Wismutoxychlorid, basisches Bleicarbonat, Bariumsulfat, metalloxidbeschichteter Glimmer und Metallflocken.

Zur Herstellung der erfindungsgemäßen Mischungen wird eine Dispersion des plättchenförmigen Substrats mit einer Dispersion des sphärischen Materials gut vermischt und es wird dann die Feststoffmischung durch Filtration, ggf. Waschen und anschließendes Trocknen gewonnen. Von entscheidender Bedeutung ist dabei, daß die Ausgangsmaterialien in gut dispergierter Form vorliegen. Dazu kann es vorteilhaft sein, die erfindungsgemäße Zumischung von sphärischen Teilchen unmittelbar an die Herstellung des jeweiligen plättchenförmigen Substrats anzuschließen solange dieses noch in dispergierter Form vorliegt.

Ein bereits in trockener und daher bereits agglomerierter Form vorliegendes Substrat kann jedoch z.B. durch Einrühren in ein Dispergiermittel und durch eine Ultraschallbehandlung sehr gut dispergiert werden. Vorzugsweise wird eine solche Behandlung für eine Zeit von etwa 1-15 min durchgeführt und vorzugsweise schon in Gegenwart der sphärischen Teilchen.

Die Art des Dispergiermittels ist an sich unkritisch. Vorzugsweise wird Wasser oder ein mit Wasser mischbares Lösungsmittel verwendet, wobei man sich von Fall zu Fall auch danach richtet, in welchem Dispergiermittel die sphärischen Teilchen vorliegen. Käufliche monodisperse SiO₂-, TiO₂- oder ZrO₂-Teilchen sind häufig in Ethylenglykol dispergiert. Andere Alkohole, wie z. B. Glycerin, Propanol oder Butanol, können jedoch mit gleich gutem Effekt eingesetzt werden.

Die Konzentration des plättchenförmigen Substrats und des sphärischen Materials in der Dispersion ist nur von untergeordneter Bedeutung für das Erreichen des erfindungsgemäßen Effekts. Vorwiegend aus Wirtschaftlichkeitserwägungen wird man die Feststoffkonzentration im Bereich von etwa 1-50 Gew.% einstellen.

Außer mit den sphärischen Teilchen kann das plättchenförmige Substrat selbstverständlich mit weiteren Stoffen gemischt oder beschichtet werden. So können beispielsweise Farbpigmente zugemischt werden oder das Substrat kann mit farblosen oder farbigen Metalloxiden beschichtet werden. Durch solche weitere Zusätze bzw. Behandlungsverfahren, die alle an sich bekannt sind, wird die Weichheit und das Dispergierverhalten des Substrats in der Regel nicht negativ beeinflußt.

Nach Beendigung des Mischvorgangs wird das plättchenförmige Substrat aus der Dispersion auf übliche Weise abgetrennt, z. B. durch Filtration, wird ggf. noch gewaschen und getrocknet und ist dann unmittelbar einsatzbereit. Gegebenenfalls kann die Pigmentmischung auch geglüht werden, ohne daß der Effekt darunter leidet.

Die Mischungen sind vielfältig sowohl in technischen als auch kosmetischen Formulierungen wie z.B. Druckfarben, Kunststoffmischungen, Hautpuder und ähnlichem einsetzbar.

### Beispiel 1

50 ml einer 10%igen Dispersion von Iriodin® 504 (plättchenförmiges Perlglanzpigment auf Basis Glimmer/Fe₂O₃ der E. Merck, Darmstadt) in Ethylenglykol werden etwa 5 min mit Ultraschall behandelt und unter weiterer Beschallung innerhalb von 10 min mit 50 ml einer 10%igen Dispersion von monodispersem SiO₂ einer Teilchengröße von etwa 300 nm versetzt. Nach weiteren 5 min Ultraschallbehandlung wird das Produkt abfiltriert und 2 h bei 100 °C getrocknet. Man erhält ein Pigment mit sehr weichem Hautfeeling.

### Beispiel 2

Es wird analog Beispiel 1 gearbeitet, wobei jedoch als plättchenförmiges Substrat Iriodin® 225 (Perlglanzpigment der E. merck, Darmstadt, auf Basis von Glimmer/TiO₂) und als sphärische Teilchen monodisperses TiO₂ (P112F des Instituto Guido Donegamii, Novara, Italien) eingesetzt wird.

### Beispiel 3

Analog Beispiel 1 wird Iriodin® 225 mit monodispersem SiO₂ einer Teilchengröße von 200-250 nm umgesetzt.

### Beispiel 4

Zu einer Suspension von 10 g Iriodin® 100 (silberfarbenes Perlglanzpigment auf Basis Glimmer/TiO₂ der E. Merck, Darmstadt) in 200 ml Wasser werden innerhalb von 50 min 200 ml einer wässerigen Suspension von 4,75 g LiChrospher® (poröses sphärisches Kieselgel mit einer mittleren Teilchengröße von 5 µm) gegeben, wobei mit Hilfe von NaOH der pH-Wert bei 8 gehalten wird. Nach 2,5stündigem Nachrühren wird abfiltriert, gewaschen und getrocknet.

### Beispiel 5

Analog Beispiel 4 wird Glimmer einer Teilchengröße von 1-50 µm mit LiChrospher® umgesetzt.

### Beispiel 6

210 ml einer Suspension von monodispersem SiO₂ einer Teilchengröße von 475 nm mit einem Gehalt von 2,35 g SiO₂/100 ml in einem Wasser/Ethanol/NH₃-Gemisch (32,2 : 64,2 : 3,6-Gewichtsteile) werden mit 290 ml Wasser, 3 Tropfen eines Tensids und 0,33 g Gelatine versetzt und 15 min bei Raumtemperatur gerührt. Nach 30minütiger Behandlung mit Ultraschall wird die Suspension innerhalb einer halben Stunde zu einer Suspension von 50 g Iriodin® 219 (Perlglanzpigment auf Basis Glimmer /TiO₂ der E. Merck, Darmstadt) in 500 ml Wasser gegeben. Danach wird mit 10%iger wässeriger Salzsäure auf pH 6 eingestellt und es wird eine Lösung von 8,67 g CrCl₃ . 6 H₂O in 500 ml Wasser innerhalb von 50 min zudosiert, wobei der pH-Wert mit 5%iger wässeriger Natronlauge bei 6,0 gehalten wird. Danach filtriert man das Produkt ab, wäscht mit Wasser chloridfrei, trocknet und glüht bei 900 °C. Sowohl das getrocknete als auch das geglühte Pigment zeichnen sich durch ihre hohe Weichheit aus.

### Beispiel 7

Man arbeitet analog Beispiel 6, setzt jedoch anstelle von CrCl₃ . 6 H₂O 7 g AlCl₃ . 6 H₂O ein.

### Beispiel 8

Eine Suspension von 5 g Iriodin® 504 in 40,5 ml Ethylenglykol wird 5 min mit Ultraschall behandelt und dann unter Rühren mit 25,2 ml einer Suspension von monodispersem SiO₂ einer Teilchengröße von 475 nm in Ethylenglykol (SiO₂-Gehalt: 12 g/l) versetzt. Nach erneuter Ultraschallbehandlung wird das Produkt abgetrennt und bei 110 °C getrocknet.

### Beispiel 9

Eine Suspension von 5 g Iriodin® 504 in 22,5 ml Glycerin und 22,5 ml Isopropanol wird analog Beispiel 8 mit 4,8 ml monodispersem SiO₂ in Glycerin (Teilchengröße 475 nm; SiO₂-Gehalt 62,5 g/l) behandelt.

### Beispiel 10

Eine Suspension von 5 g Iriodin® 504 in 45 g n-Butanol wird analog Beispiel 8 mit 9 ml monodispersem SiO₂ in n-Butanol (Teilchengröße 475 nm; SiO₂-Gehalt 33,3 g/l) behandelt.

### Beispiel 11

Eine Suspension von 5 g Iriodin® 504 in 45 ml Wasser wird analog Beispiel 8 mit 14,4 ml monodispersem SiO₂ in Wasser (Teilchengröße 475 nm; SiO₂-Gehalt 20,8 g/l) behandelt.

### Beispiel 12

Man arbeitet analog Beispiel 11, setzt jedoch 5 g Iriodin® 225 ein.

### Beispiel 13

Man arbeitet analog Beispiel 10, setzt jedoch 5 g Iriodin® 225 ein.

### Beispiel 14

Man arbeitet analog Beispiel 9, setzt jedoch 5 g Iriodin® 225 ein.

### Beispiel 15

Man arbeitet analog Beispiel 8, setzt jedoch 5 g Iriodin® 225 ein.

### Beispiel 16

100 g Glimmer einer Teilchengröße von etwa 10-50 µm werden nach dem Verfahren der DE-PS 20 09 566 unter Verwendung von 250 ml wässeriger TiCl₄-Lösung (336 g TiCl₄/l) mit TiO₂ beschichtet. Nach Abkühlen der Suspension auf 30 °C gibt man innerhalb einer Stunde bei einem pH-Wert von 4,0 235,3 ml einer Suspension von monodispersem SiO₂ (Teilchengröße 340-400 nm; SiO₂-Gehalt 2,31 g/l) in einem Wasser/Ethanol/NH₃-Gemisch (32,2 : 64,2 : 3,6 Gewichtsteilen) zu, rührt noch 15 min nach, trennt das Produkt ab, wäscht es mit Wasser chloridfrei, trocknet und glüht 30 min bei 800 °C.

## Patentansprüche

1. Deagglomerierte und gut dispergierbare plättchenförmige Substrate, gekennzeichnet durch einen Gehalt von mindestens 0,5 Gew.% sphärischen Teilchen, wobei das Verhältnis mittlerer Durchmesser der plättchenförmigen Partikel zu mittlerem Durchmesser der sphärischen Teilchen 10 : 1 oder mehr beträgt.

2. Substrat nach Anspruch 1, dadurch gekennzeichnet, daß die sphärischen Teilchen einen Durchmesser im Bereich von 0,05-50 µm besitzen.

3. Substrat nach Anspruch 2, dadurch gekennzeichnet, daß die sphärischen Teilchen eine enge Teilchengrößenverteilung besitzen.

4. Substrat nach Anspruch 1, dadurch gekennzeichnet, daß monodisperse SiO₂- und/oder TiO₂-Teilchen einer Größe im Bereich von 0,05-1,0 µm enthalten sind.

5. Verfahren zur Herstellung von deagglomerierten und gut dispergierbaren plättchenförmigen Substraten, dadurch gekennzeichnet, daß man eine Suspension eines plättchenförmigen Substrats mit einer Suspension sphärischer Teilchen, wobei das Verhältnis mittlerer Durchmesser der plättchenförmigen Partikel zu mittlerem Durchmesser der sphärischen Teilchen 10 : 1 oder mehr beträgt, vermischt und daraus das Substrat durch Abfiltrieren und Trocknen und gegebenenfalls Glühen gewinnt.

6. Verfahren nach Anspruch 5, dadurch gekennzeichnet, daß die vermischte Suspension mit Ultraschall behandelt wird.

7. Verwendung eines Substrats nach Anspruch 1 in technischen oder kosmetischen Formulierungen.

## Claims

1. Deagglomerated and readily dispersible plateletlike substrates, characterized by a content of at least 0.5% by weight of spherical particles, the ratio of the average diameter of the platelet-like particles to the average diameter of the spherical particles being 10:1 or more.

2. Substrate according to Claim 1, characterized in that the spherical particles have a diameter in the range from 0.05 to 50 µm.

3. Substrate according to Claim 2, characterized in that the spherical particles have a narrow particle size distribution.

4. Substrate according to Claim 1, characterized in that it contains monodisperse SiO₂ and/or TiO₂ particles having a size in the range from 0.05 to 1.0 µm.

5. Process for the preparation of deagglomerated and readily dispersible platelet-like substrates, characterized in that a suspension of a platelet-like substrate is mixed with a suspension of spherical particles, the ratio of the average diameter of the platelet-like particles to the average diameter of the spherical particles being 10:1 or more, and the substrate is isolated therefrom by filtering off and drying and if appropriate calcining.

6. Process according to Claim 5, characterized in that the mixed suspension is treated with ultrasound.

7. Use of a substrate according to Claim 1 in industrial or cosmetic formulations.

## Revendications

1. Substrats en paillettes désagglomérés et bien dispersables, caractérisés en ce qu'ils présentent une teneur en particules sphériques d'au moins 0,5% en poids, le rapport entre le diamètre moyen des particules lamellaires et le diamètre moyen des particules sphériques étant de 10 : 1 ou plus.

2. Substrat selon la revendication 1, caractérisé en ce que les particules sphériques présentent un diamètre de 0,05 à 50 µm.

3. Substrat selon la revendication 2, caractérisé en ce que les particules sphériques présentent une distribution granulométrique étroite.

4. Substrat selon la revendication 1, caractérisé en ce qu'il contient des particules de SiO₂ et/ou de TiO₂ monodispersé d'une dimension de 0,05 à 1,0 µm.

5. Procédé pour fabriquer des substrats en paillettes désagglomérés et bien dispersables, caractérisé en ce que l'on mélange une suspension d'un substrat en paillettes avec une suspension de particules sphériques, le rapport entre le diamètre moyen des particules lamellaires et le diamètre moyen des particules sphériques étant de 10:1 ou plus, et que l'on obtient le substrat en filtrant et en séchant et éventuellement en calcinant.

6. Procédé selon la revendication 5, caractérisé en ce que l'on traite la suspension mélangée au moyen d'ultrasons.

7. Utilisation d'un substrat selon la revendication 1 dans des formulations techniques ou cosmétiques.
